(19) Europäisches Patentamt
European Patent Office
Office européen des brevets

(11) Numéro de publication : **0 430 807 A1**

# (12) DEMANDE DE BREVET EUROPEEN

(21) Numéro de dépôt : **90403393.3**

(51) Int. Cl.$^5$ : **C07F 9/655**

(22) Date de dépôt : **29.11.90**

(30) Priorité : **02.04.90 FR 9004194**
**01.12.89 FR 8915868**

(43) Date de publication de la demande :
**05.06.91 Bulletin 91/23**

(84) Etats contractants désignés :
**AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy Les Lyon (FR)**
Inventeur : **Duhamel, Lucette**
**32 rue Jacques Boutrolles**
**F-76130 Mont Saint Aignan (FR)**
Inventeur : **Duhamel, Pierre**
**32 rue Jacques Boutrolles**
**F-76130 Mont Saint Aignan (FR)**
Inventeur : **Guillemont, Jérôme**
**7 rue Rioult**
**F-27210 Beuzeville (FR)**
Inventeur : **Poirier, Jean-Marie**
**17 impasse de la Grande Madeleine**
**F-76160 Saint Martin du Vivier (FR)**

(74) Mandataire : **Le Pennec, Magali et al**
**RHONE-POULENC RORER SA, Direction des Brevets, 20 Avenue Raymond Aron**
**F-92165 Antony Cédex (FR)**

(54) **Procédé de préparation d'halogénoacétals à partir d'énamines.**

(57) La présente invention concerne un nouveau composé de formule (IV) son procédé de préparation à partir d'halogénoacétals

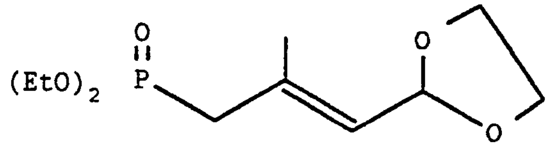

(IV)

d'aldéhydes éthyléniques par réaction avec le triéthylphosphate, et son utilisation pour la préparation du rétinal par réaction avec le β-ionylidène acétaldéhyde.

EP 0 430 807 A1

# PROCEDE DE PREPARATION D'HALOGENOACETALS A PARTIR D'ENAMINES

La présente invention concerne un nouveau procédé de préparation d'halogénoacétals d'aldéhydes éthyléniques de formule générale :

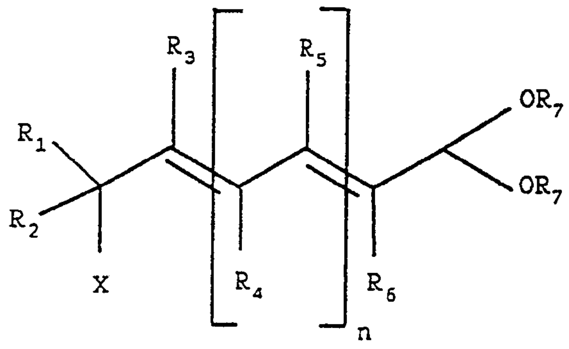

(I)

dans laquelle :

- X représente un atome d'halogène choisi parmi les atomes de chlore, de brome et d'iode
- $R_1$, $R_2$, $R_3$, $R_4$, $R_5$ et $R_6$ identiques ou différents, représentent un atome d'hydrogène ou un radical alkyle contenant 1 à 6 atomes de carbone en chaîne droite ou ramifiée, en particulier un radical méthyle ou éthyle, ou alcényle contenant 3 à 6 atomes de carbone en chaîne droite ou ramifiée et dont la double liaison est en position autre que 1-2.
- n est égal à 0, 1, 2, 3 ou 4, étant entendu que lorsque n est supérieur à 1, les différents symboles $R_4$ et $R_5$ peuvent être identiques ou différents : les symboles $R_7$ représentent chacun un radical alkyle contenant 1 à 6 atomes de carbone en chaîne droite ou ramifiée, en particulier un radical méthyle ou éthyle, ou forment ensemble un radical alkylène $R'_7$ contenant 2 à 6 atomes, de carbone en chaîne droite ou ramifiée, en particulier un radical $—CH_2\text{-}CH_2—$, et éventuellement substitué par un radical hydroxyle ou alkyloxy contenant 1 à 4 atomes de carbone.

Il est précisé que le terme halogène tel qu'utilisé ci-après englobe uniquement chlore, brome et iode.

Les halogénoacétals d'aldéhydes éthyléniques sont des composés organiques particulièrement utiles comme intermédiaires en synthèse organique. C'est ainsi qu'ils peuvent être utilisés pour introduire un motif aldéhydique α, β-éthylénique sur un reste mono ou polyénique par réaction avec une sulfone polyénique en présence d'un agent alcalin selon le procédé général décrit dans le brevet anglais 1396622, la sulfone résultant de cette condensation étant ensuite désulfonée avec formation d'une double liaison supplémentaire.

Il est connu que les -halogénoacétals d'aldéhydes α,β-éthyléniques peuvent être préparés par halogénoalkylation d'un alkyloxy-1 diène-1,3 par action d'un N-halogénosuccinimide en présence d'un alcool suivant le procédé décrit par S.M. MAKIN et coll., J. Gen. Chem. URSS, 32, 1088 (1962). Mais ce procédé a pour inconvénient la difficulté d'accès aux éthers diéthyléniques de départ ; ceux-ci sont en effet généralement préparés par traitement d'acétals d'aldéhydes α,β- ou β,γ-éthyléniques à haute température en présence de catalyseurs, les matières premières utilisées étant elles-mêmes de synthèse difficile. Bien que la méthode de MAKIN soit également applicable à la synthèse d'ω-halogénoacétals d'aldéhydes comportant un système de doubles liaisons conjuguées, la préparation de tels produits par cette méthode présente de très grandes difficultés du fait de l'accès difficile aux matières premières nécessaires.

Il est aussi connu dans le brevet US 4100201 de préparer les ω-halogénoacétals d'aldéhydes éthyléniques par halogénation d'un ester de préférence acétique correspondant à l'aldéhyde éthylénique. Cet ester est préparé selon l'article de M.J. MAGEMEYER paru dans Ind. Eng. Chem. 41, 2920 (1949) par échange entre l'aldéhyde et l'acétate d'isopropényle. Lors de la regénération de l'aldéhyde après hydrolyse de la fonction ester l'acide acétique est libéré ce qui ne permet pas de régénerer la matière première couteuse qu'est l'acétate d'isopropényle. Ce procédé est donc, comme le précédent, non économique.

Il est encore connu d'après le brevet US4087405 de préparer les ω-halogénoacétals d'aldéhydes polyéniques par réaction d'un cation halogène sur un énoxysilane. L'énoxysilane est un composé difficilement accessible d'un point de vue industriel et pas là même une matière première coûteuse.

Le procédé selon l'invention permet d'accéder aux halogénoacétals d'aldéhydes insaturés avec de bons rendements à partir de matières premières accessibles, peu couteuses et facilement recyclables.

Il a en effet été trouvé que l'on peut préparer les halogénoacétals d'aldéhydes éthyléniques de formule générale (I) par réaction d'un agent d'halogénation choisi parmi les cations halogènes $Cl^+$, $Br^+$ et $I^+$ sur une

énamine de formule générale :

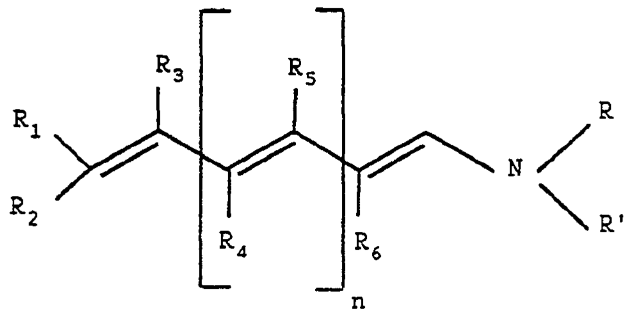

(II)

dans laquelle $R_1$, $R_2$, $R_3$, $R_4$, $R_5$ $R_6$ et n sont définis comme précédemment et R et R' représentent des radicaux alkyle contenant 1 à 6 atomes de carbone en chaîne droite ou ramifiée ou un radical alkylène ou alkenylène contenant éventuellement des hétéroatomes choisis parmi l'oxygène, le soufre et l'azote, en particulier ils peuvent former un cycle pipéridinique ou morpholinique, puis, après action de l'eau, réaction du produit obtenu sur un alcool primaire ou secondaire de formule générale $R_7OH$ dans laquelle $R_7$ est défini comme précédemment ou sur un glycol de formule générale $HO\text{-}R'_7\text{-}OH$ dans laquelle $R'_7$ est défini comme précédemment ou encore sur un orthoformiate de formule $HC(OR_7)_3$ ou mixte de formule $(O_2R_7')CH\text{-}(OR_7)$ obtenu par condensation d'un orthoformiate $HC(OR_7)_3$ et d'un glycol de formule $HOR_7'OH$.

Les cations halogènes sont connus depuis de nombreuses années, en particulier par J. AROTSKY et M.C.R. SYMONS, Quart. Rev. 16282 (1962) ; il est également connu que ces cations halogènes peuvent être mis en évidence par différentes méthodes, telles que la mesure de la conductivité et la spectrométrie de masse par exemple. De nombreux produits sont connus pour être la source de tels cations halogènes [Peter B.D. de La Marre, "Electrophilic Halogenation", Cambridge Chemistry Texts, 1976]. Une première classe de produits utilisables comme source de cations halogènes est constituée par les halogènes eux mêmes et les produits dans lesquels un atome d'halogène est lié par liaison covalente à un atome d'azote d'oxygène, de phosphore ou de soufre ; à titre d'exemple on peut citer en particulier les hypohalogénites alcalins, les hypohalogénites organiques, les N-halogénoamines, les N-halogénoamides, les N-halogéno-carbo-imides, les N-halogéno-sulfo-imides, les N-halogéno-carbo-sulfo-imides les N-halogéno-hydantoines, les N-halogéno-triazoles et N-benzo-triazoles, les oxychlorures de phosphore, le chlorure de thionyle. Une seconde classe de produits utilisables comme source de cations halogènes est constituée par les composés résultant de l'addition d'halogène moléculaire sur un halogénure d'ammonium quaternaire aliphatique, aromatique ou cyclique ou sur un halogénure aromatique. Une troisième classe de produits utilisables comme source de cations halogènes est constituée par les complexes formés par action d'un halogène moléculaire sur un amide aliphatique ou cyclique.

Peuvent être plus spécialement cités comme sources de cations halogènes, les hypohalogénites alcalins, les hypohalogénites organiques, en particulier les hypohalogénites d'alcools aliphatiques tertiaires saturés contenant jusqu'à 13 atomes de carbone, les N-chloro et N-bromo-succinimides, les N-bromo et N-chloro polymaléimides, les N-bromo et N-chloro caprolactames, les dichloro-1,3 et dibromo-1,3 diméthyl-5,5 hydantoines, les N-bromo et N-chloro saccharines, le chlorobenzotriazole, le N-bromo acétamide, la bromourée, la chloramine, le perbromure de phényl-triméthylammonium, l'iodure de tétrachlorotétra-n.butylammonium, l'iodure de dichloro tétra-n.butylammonium, le tribromure de tétra-n.butylammonium, le perbromure de pyridinium, le dichlorure d'iodobenzène, les oxychlorures de phosphore, le chlorure de thionyle, les complexes fournis par action du chlore, du brome ou de l'iode sur le diméthylformamide, le diméthylacétamide et la N-méthylpyrrolidone.

D'une manière générale, il est suffisant de faire réagir un cation halogène par mole d'énamine de formule générale (II), c'est-à-dire d'utiliser la quantité de produit générateur de cation halogène nécessaire pour fournir un cation halogène par mole d'énamine de formule générale (II), un excès de l'un ou de l'autre de ces réactifs pouvant toutefois être utilisé sans inconvénient. Après la réaction entre le cation halogène et l'énamine de formule générale (II), on ajoute de l'eau, puis un excès de l'alcool de formule générale $R_7OH$, d'orhtoformiate $HC(OR_7)_3$ ou mixte ou encore du glycol de formule générale $HO\text{-}R'_7\text{-}OH$. Cette réaction permet de réaliser la transformation de l'énamine de formule générale (II) en halogénoacétal de formule générale (I), dans le même milieu réactionnel ("one pot synthesis"), sans isoler les produits intermédiaires. La température de la réaction n'est pas critique et peut être comprise par exemple entre -70 et +80°C, et de préférence entre -60 et +20°C afin d'éviter une décomposition appréciable des produits.

Lorsque l'on utilise comme source de cation halogène des halogènes moléculaires ou des composés dans

lesquels l'atome d'halogène est lié à un atome d'azote ou d'oxygène ou un composé résultant de l'addition d'un halogène moléculaire sur un halogénure d'ammonium quaternaire aliphatique aromatique ou cyclique or sur un halogénure aromatique, on opère généralement à une température comprise entre -70°C et +80°C selon la stabilité du produit utilisé comme source de cation halogène et particulièrement entre -70 et +30°C. Afin d'accélérer la vitesse de la réaction d'acétalisation, il est avantageux d'opérer en présence d'une quantité catalytique d'un acide minéral ou organique connu comme catalyseur d'acétalisation, tel que les acides chlorhydrique, sulfurique et méthanesulfonique ; cet acide peut être introduit dans le mélange réactionnel après réaction du produit générateur de cation halogène sur l'énamine de formule générale (II).

Lorsque l'on condense l'halogène cationique dont la source a pour formule AX sur l'énamine de formule (II) il se forme intermédiairement un sel d'iminium de formule générale (III) qui est un produit nouveau.

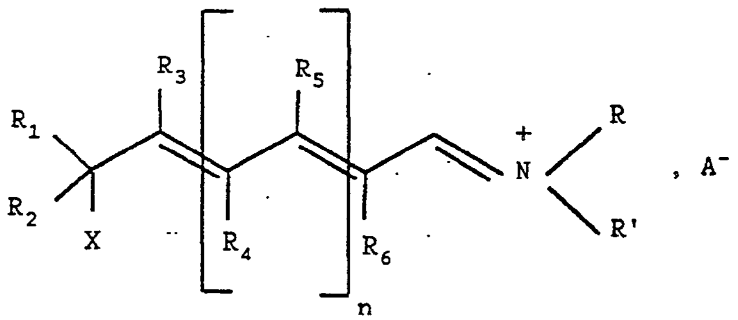

(III)

formule dans laquelle X, $R_1$, $R_2$, $R_3$, $R_4$, $R_5$, $R_6$, R, R' et n ont la même signification que dans la formule (I) et (II), $A^-$ représente l'anion lié au cation halogène dans la source de cation halogène. $A^-$ peut ainsi représenter: un halogène X si la source de cation halogène est l'halogène moléculaire, un groupe par exemple oxydichlorure de phosphore ($POCl_2^-$), un groupe par exemple monochlorure de thionyle ($SOCl^-$).

Il peut également se former à côté du produit (III) un produit isomère (IIIa) de formule suivante :

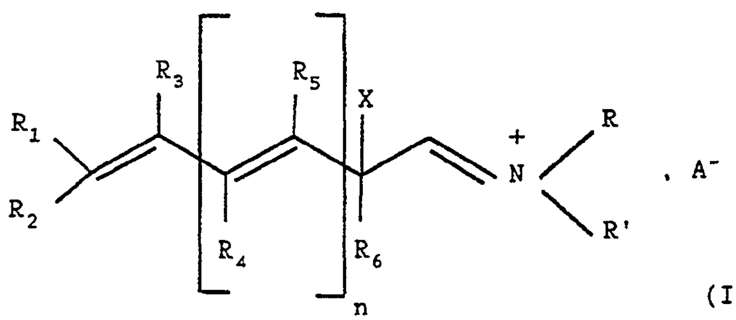

(IIIa)

qui sera facilement converti en produit de formule (III) simplement par augmentation de la température du milieu réactionnel.

Les énamines de formule (II) utilisées comme matières premières sont préparées selon S.HUNIG et H. KAHANEK, Chem. Ber. 1957, 90, 238, N. F. FIRRELL, P.W. HICKMOTT et B.J. HOPKINS J.C.S. (B) 1971, 351, E.DEMOLE, C. DEMOLE ET P. ENGGIST Helv. Chim. Act. 1976, 59, 737 par mise en contact de l'amine de formule

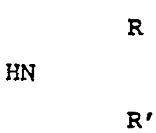

avec l'aldéhyde polyénique en présence d'un déshydratant dans un solvant aliphatique ou aromatique inerte vis-à-vis des réactifs. Les réactifs sont mis en présence à la température ambiante. Après réaction, le déshydradant est éliminé, le solvant est éliminé par distillation et l'énamine est obtenue par distillation.

Après halogénation et acétalisation on récupère d'une part l'halogénoacétal éthylénique de formule (I) et l'amine peut être facilement recyclée.

Les produits obtenus de formule (I) (n = O) sont utilisés notamment pour la synthèse de la vitamine E ou du rétinal par condensation directement avec une sulfone contenant 15 atomes de carbone selon le procédé décrit dans le brevet GB 1396622 ou par passage par un intermédiaire phosphonique IV qui est ensuite condensé en présence d'une base sur le β-ionylidène acétaldéhyde. L'intermédiaire phosphonique (IV) est préparé par condensation du dérivé de formule (I), (n = O) avec un alkylphosphite.

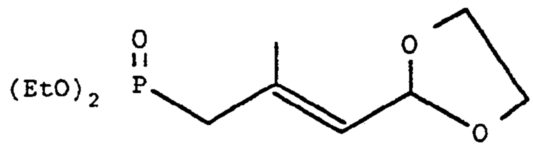

(IV)

Le procédé selon la présente invention convient particulièrement bien pour la préparation du dioxolanne du méthyl-3 bromo-4 butène-2 al-1 à partir du morpholino-1 méthyl-3 butadiène-1,3.

Les exemples suivants, donnés à titre non limitatif, montrent comment le procédé selon la présente invention peut être mis en pratique.

## EXEMPLE 1

### Préparation du méthyl-3 morpholino-1 butadiène

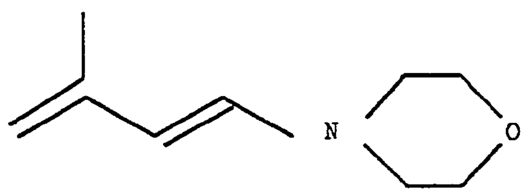

Sous atmosphère inerte, dans un erlenmeyer de 100 ml muni d'une ampoule à brome, on introduit 43,5 mmol de carbonate de potassium anhydre (6 g) et 308 mmol de morpholine (26,8 g). Une fois le milieu réactionnel refroidi à 0°C, on ajoute goutte à goutte 150 mmol de prénal dans 20 ml de toluène. On laisse remonter la température à 20°C et l'on poursuit l'agitation pendant 4 heures. On sépare le carbonate de potassium par filtration. On élimine le toluène à la pression atmosphérique ce qui permet le cracking de l'aminal. Sous pression réduite on distille tout d'abord la morpholine en excès, puis la diénamine attendue (Eb 108-110°C sous 20 mm de Hg).

## EXEMPLE 2

### Préparation du bromo-4 méthyl-3 butène-2 al.

A 10 mmol de diénamine (1,53 g) de l'exemple 1 en solution dans 100 ml d'éther anhydre, on ajoute à une température inférieure à -60°C une solution de 10 mmol de brome (1,6 g) dans 50 ml d'éther anhydre préalablement refroidie à -70°C. Après un quart d'heure d'agitation à -70°C, on remonte en cinq minutes à +35°C. Le reflux de l'éther est maintenu pendant 15 minutes.

Pour caractériser le sel d'iminium ω-bromé, le précipité jaune est filtré à l'abri de l'humidité, puis séché au dessicateur 3 heures à température ambiante en présence de $P_4O_{10}$. Le spectre RMN proton effectué dans l'acétonitrile deutéré a permis de caractériser le sel d'iminium ω-bromé de formule :

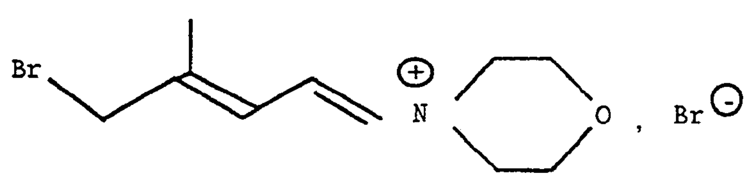

Sa formation est quantitative.
RMN $^1H,CD_3CN$ : isomère présent à 25% :
9,5 (d,1H,J = 11Hz) ; 6,7 (d,1H,J = 11Hz) ; 4,7 (s,2H) ; 4-3,7 (m,8H) ; 2,2 (s,3H).
RMN $^1H,CD_3CN$, : isomère présent à 75% ; 9,1 (d,1H,J = 11Hz) ; 7, 1 (d,1H,J = 11Hz) ; 4,3 (s,2H) ; 4-3,7 (m,8H); 2,3 (s,3H).

On procède dans le même réacteur à l'hydrolyse du sel d'iminium par 11 équivalents d'eau (2 ml) distillée. Le milieu réactionnel est agité vigoureusement à température ambiante pendant deux heures.

On vérifie la formation du prénal ω-bromé en l'isolant (formule A) :
Eluant : éther/éther de pétrole : 10/100.
Rdt : 70%. IR : 1670 (ν C=0) ; 1640 (ν C=C)

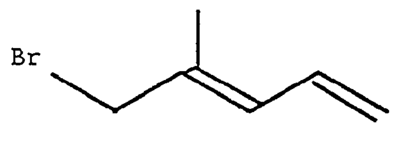

A

Si, dans le réacteur, on procède à l'hydrolyse par 11 1équivalents d'eau (2 ml) à -70°C, 30 minutes après l'addition du brome, on isole un produit brut (Rdt : 88%), constitué d'aldéhydes isomères A et B (A/B = 63/37)

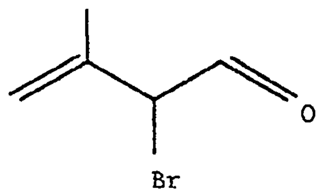

B

## EXEMPLE 3

Préparation du dioxolanne du bromo-4 méthyl-3 butène-2 al

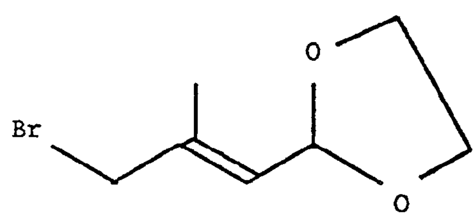

Sur le milieu réactionnel obtenu selon l'exemple 2, on introduit successivement 20 ml d'un orthoformiate mixte l'éthylènedioxyméthoxy méthane (19 eq) et 4 gouttes d'une solution de chlorure d'hydrogène/éthylène glycol à 12% en poids. Il est nécessaire de maintenir le mélange réactionnel sous agitation pendant une heure avant d'observer en CCM la disparition complète de l'aldéhyde.

La neutralisation du milieu est réalisée par addition de méthylate de sodium solide (environ 100 mg). On sèche sur sulfate de magnésium et on concentre. On récupère 1,5 g de produit brut. Après purification par chromatographie éclair sur support de silice, on isole 49% d'acétal ω-bromé.

## EXEMPLE 4

Préparation du phosphonate du dioxolanne du méthyl-3 butène-2 al

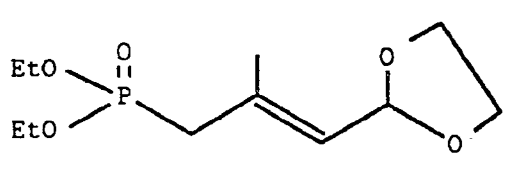

Dans un bicol de 25 ml, muni d'un thermomètre et d'un réfrigérant, on introduit successivement 0,83 g (5 mmol) de triéthyle phosphite dans 5 ml de toluène sec et 1 g (4,83 mmol) de bromo dioxolanne de l'exemple 3 dans 10 ml de toluène.

Le milieu réactionnel est chauffé au reflux du toluène durant 15 heures.

Après disparition totale en CCM du spot de l'acétal bromé et élimination du solvant, le phosphonate est purifié par distillation sous pression réduite (point d'ébullition : 110-120°C/0,3 mmHg), rendement : 80%.

EXEMPLE 5

Préparation du rétinal

Sous argon, dans un bicol de 25 ml, on introduit 0,7 mmol de phosphonate de l'exemple 4 (0,185 g) en solution dans 10 ml de THF. On ajoute par petites fractions à -70°C, 1 mmol de tertiobutylate de potassium (0,112 g). On poursuit l'agitation 90 minutes à -70°C. On ajoute ensuite, 0,5 mmol de β-ionylidène acétaldéhyde (0,109 g) en solution dans un ml de THF. La température est maintenue 15 minutes à -70°C puis, 1 heure à -20°C.

L'hydrolyse du milieu réactionnel est effectuée à -20°C avec une solution d'acide chlorhydrique 3 N(5 ml). Après un quart d'heure d'agitation, on extrait la phase aqueuse à l'éther (5 fois 15 ml), sèche sur sulfate de magnésium et concentre. Le produit brut est purifié par chromatographie éclair (éluant éther/éther de pétrole 10/100). Le rendement en rétinal est de 66%.

## Revendications

1. Composé répondant à la formule (IV)

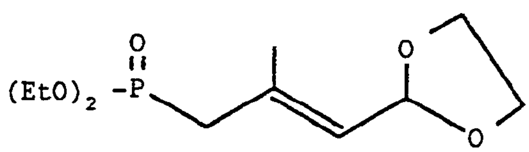

(IV)

2. Procédé de préparation du composé (IV) caractérisé en ce que l'on met en présence l'halogénoacétal de formule (I)

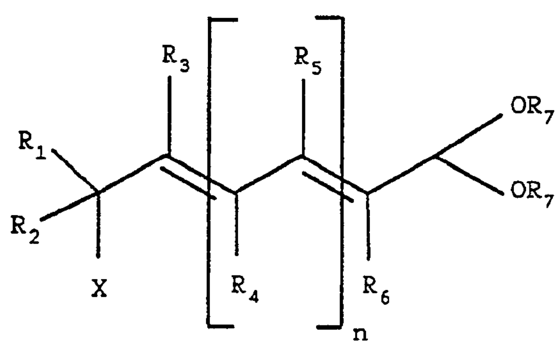

(I)

dans laquelle :
- X représente le brome
- $R_1 = R_2 = R_6$ représente l'hydrogène
- $R_3$ représente un radical méthyl
- les symboles $R_7$ forment ensemble un radical éthylène avec le triéthylphosphate dans un solvant organique.

3. Utilisation du composé de formule (IV) pour la préparation du rétinal caractérisé en ce qu'on le fait réagir en présence d'une base sur le β-ionylidène acétaldéhyde, puis on élimine le groupe protecteur au moyen

d'un acide fort dans un solvant organique.

Office européen des brevets

# RAPPORT DE RECHERCHE EUROPEENNE

Numero de la demande

EP 90 40 3393

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 68, 26 février 1968, page 3869, abrégé no. 39729y, Columbus, Ohio, US; V.V. MOSKVA et al.: "Phosphinic and phosphinous acids. XXXIV. Phosphorylated 1,3-dioxolanes", & TR. KAZAN. KHIM. TEKKNOL. INST., no. 34, 273-7(1965)<br>----- | | C 07 F 9/655 |

DOMAINES TECHNIQUES RECHERCHES (Int. Cl.5)

C 07 F 9/00
C 07 C 403/00

Le présent rapport a été établi pour toutes les revendications

| Lieu de la recherche | Date d'achèvement de la recherche | Examinateur |
|---|---|---|
| LA HAYE | 24-01-1991 | OUSSET J-B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)